# EUROPÄISCHE PATENTSCHRIFT

(11) **EP 3 873 430 B1**
(45) Veröffentlichungstag und Bekanntmachung des Hinweises auf die Patenterteilung: **24.06.2026**
(21) Anmeldenummer: 19798231.7
(22) Anmeldetag: 31.10.2019
(51) Int. Cl.: A61Q 5/12, A61K 8/58, A61Q 5/06, A61Q 5/00, A61K 8/898, A61K 8/892, A61K 8/891

(54) **WIRKSTOFFZUSAMMENSETZUNG ZUR PFLEGE VON HUMANHAAREN**
COMPOSITION OF ACTIVE INGREDIENTS, FOR CARE OF HUMAN HAIR
COMPOSITION DE PRINCIPES ACTIFS POUR LE SOIN DES CHEVEUX HUMAINS

(30) Priorität: 31.10.2018 DE 102018127239
(43) Veröffentlichungstag der Anmeldung: 08.09.2021
(73) Patentinhaber: Henkel AG & Co. KGaA, 40589 Düsseldorf (DE)
(72) Erfinder: KROHN, Rene, 22848 Norderstedt (DE); SCHULZE ZUR WIESCHE, Erik, 33619 Bielefeld (DE); LECHNER, Torsten, 40764 Langenfeld (DE)
(86) Internationale Anmeldenummer: PCT/EP2019/079791
(87) Internationale Veröffentlichungsnummer: WO 2020/089375

(56) Entgegenhaltungen:
- EP-A2- 1 767 187
- FR-A1- 2 966 356
- US-A1- 2017 189 319
- DATABASE GNPD [online] MINTEL; 29 November 2017 (2017-11-29), ANONYMOUS: "Pressed Powder N Refill", XP055661133, retrieved from www.gnpd.com Database accession no. 5277679
- DATABASE GNPD [online] MINTEL; 16 April 2018 (2018-04-16), ANONYMOUS: "Intra-Cylane Fortifying Cream", XP055661162, retrieved from www.gnpd.com Database accession no. 5579027
- GRANT INDUSTRIES, INTERNET CITATION, 1 January 2004 (2004-01-01), XP002387469, Retrieved from the Internet <URL:http://www.grantinc.com/Personal_Care/SpecShts/Hair_Care/hair.htm> [retrieved on 20060627]

## Beschreibung

Die vorliegende Erfindung betrifft kosmetische Mittel zur Behandlung eines keratinischen Materials, wobei das Mittel eine organische Siliciumverbindung und ein Polyorganosiloxan umfasst, sowie die Verwendung des kosmetischen Mittels.

Die äußere Beanspruchung der Haare durch chemische Stoffe aus einer Vielzahl unterschiedlicher Quellen stellt die Entwicklung kosmetischer Pflegeprodukte vor Herausforderungen. Luft- und Wasserverunreinigungen wirken sich nachteilig auf Haut und Haare aus. Zu den wichtigsten Luftschadstoffen gehören polycyclische aromatische Kohlenwasserstoffe, flüchtige organische Verbindungen, Stickoxide (NOx), Partikel und Zigarettenrauch. Die Wirkung verschiedener Luftschadstoffe kann in Gegenwart anderer Luftschadstoffe und unter Einwirkung von UV-Strahlung verstärkt werden.

Es ist bekannt, dass die Toxizität von gasförmigen Schadstoffen der Luft, wie Schwefeldioxid, Ozon und Stickoxiden, insbesondere mit ihrer Initiatoraktivität für freie Radikale zusammenhängt, die bei Lebewesen Schäden verursachen. Freie Radikale sind Stoffwechselprodukte, die auch natürlicherweise im Körper vorkommen. In grosser Menge können freie Radikale Irritationen und Entzündungen begünstigen und den Prozess der Alterung beschleunigen. In dem Fall spricht man von "oxidativem Schaden". Freie Radikale können auch eine Haarschädigung bewirken, die beispielsweise als Verringerung des Glanzes sowie des Griffs und/oder des Verblassens der Haarfarbe sichtbar wird.

Partikel sind eine komplexe Mischung, die Metalle, Mineralien, organische Toxine und/oder biologische Materialien enthalten. Auch sie können die Bildung von freien Radikalen begünstigen.

Ferner sind oft wechselnde Konsumentenwünsche hinsichtlich einer bestimmten Beschaffenheit der Haare mit einer wiederkehrenden chemischen Beanspruchung der Haare verbunden. Beispielsweise beanspruchen Haarfärbungen die Haare, aufgrund dessen eine besondere, intensive Pflege nötig sein kann. Besonders wünschenswert ist eine Pflege, die das Auswaschen von oxidativen Farbstoffen minimiert.

Im Stand der Technik werden siliciumorganische Verbindungen aus der Gruppe der Silane beschrieben, die mindestens eine Hydroxygruppe und/oder hydrolysierbare Gruppe umfassen. Aufgrund der Anwesenheit der Hydroxygruppen und/oder hydrolysierbaren Gruppen handelt es sich bei den Silanen um reaktive Substanzen, die in Gegenwart von Wasser hydrolysieren bzw. oligomerisieren oder polymerisieren. Die durch Anwesenheit des Wassers initiierte Oligomerisierung oder Polymerisierung der Silane führt bei Anwendung auf einem keratinischen Material letztendlich zur Ausbildung eines Films, der eine Schutzwirkung entfalten kann.

Es besteht Bedarf an einem Produkt, das das aufgrund von Haarbehandlungen oder aufgrund von Luft- und Wasserverunreinigungen beanspruchte keratinische Material, insbesondere Haare, besonders gut pflegt.

Die der vorliegenden Erfindung zugrunde liegende Aufgabe besteht in der Bereitstellung eines Produkts mit einer herausragenden Pflege- und/oder Schutzwirkung.

Diese Aufgabe wird gelöst durch ein kosmetisches Mittel zur Behandlung eines keratinischen Materials, umfassend
a) mindestens eine organische Siliciumverbindung, wobei die mindestens eine organische Siliciumverbindung eine Verbindung der Formel (I) und mindestens eine organische Siliciumverbindung der Formel (IV) enthält,
   wobei in der organischen Siliciumverbindung der Formel (I)

      R₁R₂N-L-Si(OR₃)a(R₄)b (I),

      - R₁, R₂ beide für ein Wasserstoffatom stehen,
      - L für eine lineare, zweibindige C₁-C₆-Alkylengruppe, bevorzugt für eine Propylengruppe (-CH₂-CH₂-CH₂-) oder für eine Ethylengruppe (-CH₂-CH₂-), steht,
      - R₃, R₄ unabhängig voneinander für eine Methylgruppe oder für eine Ethylgruppe stehen,
      - a für die Zahl 3 steht und
      - b für die Zahl 0 steht, und
   wobei in der organischen Siliciumverbindung der Formel (IV)

      R₉Si(OR₁₀)k(R₁₁)m (IV),

      - R₉ für eine C₁-C₁₂-Alkylgruppe steht,
      - R₁₀ für ein Wasserstoffatom oder eine C₁-C₆-Alkylgruppe steht,
      - R11 für eine C1-C6-Alkylgruppe steht
      - k für eine ganze Zahl von 1 bis 3 steht, und
      - m für die ganze Zahl 3 - k steht , und
b) mindestens ein Polyorganosiloxan, umfassend PCA (Pyrrolidoncarbonsäure) Dimethicone/Amodimethicone (INCI).

Unter einem keratinischen Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter einem keratinischen Material das menschliche Haar, die menschliche Haut und menschliche Nägel, insbesondere Finger- und Fußnägel, verstanden. Ganz besonders bevorzugt wird unter keratinischem Material das menschliche Haar, insbesondere Kopf- und/oder Barthaare, verstanden.

Als ersten erfindungswesentlichen Bestandteil enthält das kosmetische Mittel zur Behandlung eines keratinischen Materials mindestens eine ausgewählte organische Siliciumverbindung. Unter den Begriff "organische Siliciumverbindung" soll kein Polydimethylsiloxan fallen. Dies soll sicher stellen, dass die Komponenten a) und b) in jedem Fall zwei sich unterscheidende Komponenten darstellen.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist. Die organische Siliciumverbindungen sind Verbindungen, die ein bis drei Siliciumatome enthalten.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die Wasserstoff-Atome ganz oder teilweise durch organische Gruppen wie beispielsweise (substituierte) Alkylgruppen und/oder Alkoxygruppen ersetzt. In den organischen Silanen kann auch ein Teil der Wasserstoffatome durch Hydroxygruppen ersetzt sein.

Das Mittel zur Behandlung eines keratinischen Materials enthält mindestens eine ausgewählte organischen Siliciumverbindung, die bevorzugt aus Silanen mit einem, zwei oder drei Siliciumatomen ausgewählt ist, wobei die organische Siliciumverbindung eine oder mehrere Hydroxylgruppen oder hydrolysierbare Gruppen pro Molekül umfasst.

Das Mittel zur Behandlung eines keratinischen Materials enthält mindestens eine organische Siliciumverbindung der Formel (I),

R₁R₂N-L-Si(OR₃)a(R₄)b (I),

wobei
- R₁, R₂ beide für ein Wasserstoffatom stehen,
- L für eine lineare, zweibindige C₁-C₆-Alkylengruppe, bevorzugt für eine Propylengruppe (-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen,
- a für die Zahl 3 steht und
- b für die Zahl 0 steht

Die Substituenten R₁, R₂, R₃, R₄, und L in den Verbindungen der Formel (I) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Amino-C₁-C₆-alkyl-gruppe sind die Aminomethylgruppe, die 2-Aminoethylgruppe, die 3-Aminopropylgruppe. Die 2-Aminoethylgruppe ist besonders bevorzugt.

Beispiele für eine lineare zweibindige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweibindige Alkylengruppen auch verzweigt sein. Beispiele für verzweigte, zweibindige C₃-C₂₀-Alkylengruppen sind (-CH₂-CH(CH₃)-) und (-CH₂-CH(CH₃)-CH₂-).

In den organischen Siliciumverbindungen der Formel (I)

R₁R₂N-L-Si(OR₃)a(R₄)b (I),

stehen die Reste R₁ und R₂ unabhängig voneinander für ein Wasserstoffatom oder eine C₁-C₆-Alkylgruppe. Ganz besonders bevorzugt stehen die Reste R₁ und R₂ beide für ein Wasserstoffatom.

Im Mittelteil der organischen Siliciumverbindung befindet sich die Struktureinheit oder der Linker -L- der für eine lineare oder verzweigte, zweibindige C₁-C₂₀-Alkylengruppe steht.

Bevorzugt steht -L- für eine lineare, zweibindige C₁-C₂₀-Alkylengruppe. Weiter bevorzugt steht -L- für eine lineare zweibindige C₁-C₆-Alkylengruppe. Besonders bevorzugt steht -L- für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt steht L für eine Propylengruppe (-CH₂-CH₂-CH₂-).

Die organischen Siliciumverbindungen der Formel (I)

R₁R₂N-L-Si(OR₃)a(R₄)b (I),

tragen jeweils ein einem Ende die Silicium-haltige Gruppierung -Si(OR₃)ₐ(R₄)_{b}.

In der endständigen Struktureinheit -Si(OR₃)ₐ(R₄)_{b} steht der Rest R₃ für ein Wasserstoffatom oder eine C₁-C₆-Alkylgruppe, und der Rest R₄ steht für eine C₁-C₆-Alkylgruppe. Besonders bevorzugt stehen R₃ und R₄ unabhängig voneinander für eine Methylgruppe oder eine Ethylgruppe.

Hierbei steht a für eine ganze Zahl von 1 bis 3, und b steht für die ganze Zahl 3 - a. Wenn a für die Zahl 3 steht, dann ist b gleich 0. Wenn a für die Zahl 2 steht, dann ist b gleich 1. Wenn a für die Zahl 1 steht, dann ist b gleich 2.

Der beste Schutz vor den negativen Auswirkungen von Wasser- und/oder Luftverschmutzungen ("Anti-Pollution"-Wirkung) und die beste Pflege von beanspruchtem Haar konnte erhalten werden, wenn das Mittel zur Behandlung eines keratinischen Materials mindestens eine organische Siliciumverbindung der Formel (I) enthält, in welcher die Reste R₃, R₄ unabhängig voneinander für eine Methylgruppe oder für eine Ethylgruppe stehen.

Besonders gut geeignete organische Siliciumverbindungen der Formel (I) sind - (3-Aminopropyl)triethoxysilan 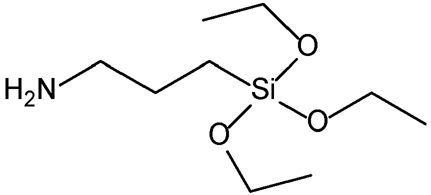 - (3-Aminopropyl)trimethoxysilan 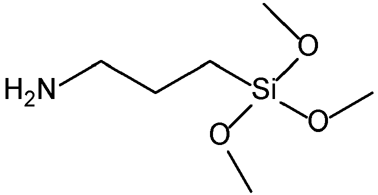 -1-(3-Aminopropyl)silantriol 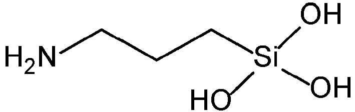 - (2-Aminoethyl)triethoxysilan 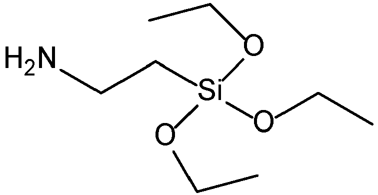 - (2-Aminoethyl)trimethoxysilan 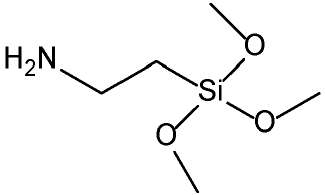 und -1-(2-Aminoethyl)silantriol

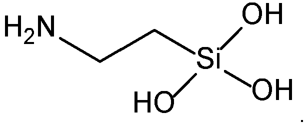

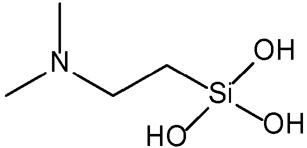

Die vorgenannten organische Siliciumverbindung der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erhältlich.

Im Rahmen einer weiteren Ausführungsform kann das Mittel zur Behandlung eines keratinischen Materials ferner mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II)

enthalten.

Die siliciumorganischen Verbindungen der Formel (II) tragen jeweils an ihren beiden Enden die Siliciumhaltigen Gruppierungen (R₅O)_{c}(R₆)_{d}Si- und -Si(R₆')_{d'}(OR₅')_{c'}.

Im Mittelteil des Moleküls der Formel (II) befinden sich die Gruppierungen -(A)ₑ- und -[NR₇-(A')]_{f}- und -[O-(A")]_{g}- und -[NR₈-(A‴)]ₕ-. Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält eine organischen Siliciumverbindung der Formel (II) mindestens eine Gruppierung aus der Gruppe aus -(A)- und -[NR₇-(A')]- und -[O-(A")]- und -[NR₈-(A‴)]-.

In den beiden endständigen Struktureinheiten (R₅O)_{c}(R₆)_{d}Si- und - Si(R₆')_{d'}(OR₅')_{c'} stehen die Reste R5, R5', R5" unabhängig voneinander für ein Wasserstoffatom oder für eine C₁-C₆-Alkylgruppe. Die Reste R6, R6' und R6" stehen unabhängig voneinander für eine C₁-C₆-Alkylgruppe.

Hierbei steht c für eine ganze Zahl von 1 bis 3, und d steht für die ganze Zahl 3 - c. Wenn c für die Zahl 3 steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' für die Zahl 3 steht, dann ist d' gleich 0. Wenn c' für die Zahl 2 steht, dann ist d' gleich 1. Wenn c' für die Zahl 1 steht, dann ist d' gleich 2.

Eine sehr hohe Anti-Pollution-Wirkung des Mittels zur Behandlung eines keratinischen Materials konnte erhalten werden, wenn die Reste c und c' beide für die Zahl 3 stehen. In diesem Fall stehen d und d' beide für die Zahl 0.

In einer weiteren bevorzugten Ausführungsform enthält das Mittel zur Behandlung eines keratinischen Materials mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die organischen Siliciumverbindung der Formel (IIa)

(R₅O)₃Si-(A)ₑ-[NR₇-(A')]_{f}-[O-(A")]_{g}-[NR₈-(A‴)]ₕ-Si(OR_{5'})₃ (IIa).

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen -(A)ₑ- und -[NR₇-(A')]_{f}- und -[O-(A")]_{g}- und -[NR₈-(A‴)]ₕ- sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erhöhung der "Anti-Pollution"-Wirkung erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die organischen Siliciumverbindung der Formel (IIb)

(R₅O)_{c}(R₆)_{d}Si-(A)-[NR₇-(A')]-Si(R₆')_{d'}(OR₅')_{c'} (IIb).

Die Reste A, A', A", A‴ und Aʺʺ stehen unabhängig voneinander für eine lineare oder verzweigte, zweibindige C₁-C₂₀-Alkylengruppe. Bevorzugt stehen die Reste A, A', A", A‴ und Aʺʺ unabhängig voneinander für eine lineare, zweibindige C₁-C₂₀-Alkylengruppe. Weiter bevorzugt stehen die Reste A, A', A", A‴ und Aʺʺ unabhängig voneinander für eine lineare zweibindige C₁-C₆-Alkylengruppe. Besonders bevorzugt stehen die Reste A, A', A", A‴ und Aʺʺ unabhängig voneinander für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt stehen die Reste A, A', A", A‴ und Aʺʺ für eine Propylengruppe (-CH₂-CH₂-CH₂-).

Wenn der Rest f für die Zahl 1 steht, dann enthält die organische Siliciumverbindung der Formel (II) eine strukturelle Gruppierung -[NR₇-(A')]-.

Wenn der Rest h für die Zahl 1 steht, dann enthält die organische Siliciumverbindung der Formel (II) eine strukturelle Gruppierung -[NR₈-(A‴)]-.

Hierbei stehen die Reste R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-Alkylgruppe oder eine Gruppierung der Formel (III)

- (Aʺʺ)-Si(R₆")_{d}"(OR₅")_{c}" (III).

Ganz besonders bevorzugt stehen die Reste R₇ und R₈ unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (III).

Wenn der Rest f für die Zahl 1 steht und der Rest h für die Zahl 0 steht, enthält die organische Siliciumverbindung die Gruppierung [NR₇-(A')], aber nicht die Gruppierung -[NR₈-(A‴)], Steht nun der Rest R7 für eine Gruppierung der Formel (III), so enthält das Mittel zur Behandlung eines keratinischen Materials eine organische Siliciumverbindung mit 3 reaktiven Silan-Gruppen.

In einer weiteren bevorzugten enthält das Mittel zur Behandlung eines keratinischen Materials mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- e und f beide für die Zahl 1 stehen,
- g und h beide für die Zahl 0 stehen,
- A und A' unabhängig voneinander für eine lineare, zweibindige C₁-C₆-Alkylengruppe stehen
   und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

In einer weiteren bevorzugten Ausführungsform enthält das Mittel zur Behandlung eines keratinischen Materials mindestens eine organische Siliciumverbindung der Formel (II), wobei
- e und f beide für die Zahl 1 stehen,
- g und h beide für die Zahl 0 stehen,
- A und A' unabhängig voneinander für eine Methylengruppe (-CH₂-), eine Ethylengruppe (-CH₂-CH₂-) oder eine Propylengruppe (-CH₂-CH₂-CH₂) stehen,
   und
- R₇ für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

Zur Lösung der Aufgabenstellung gut geeignete organische Siliciumverbindungen der Formel (II) sind - 3-(Trimethoxysilyl)-N-[3-(trimethoxysilyl)propyl]-1-propanamin 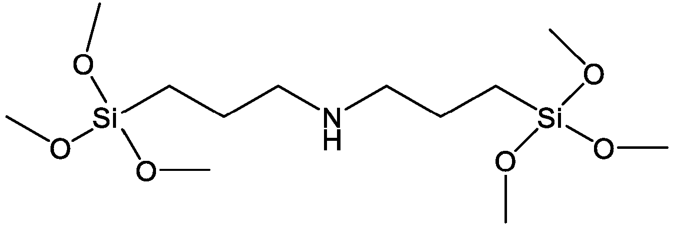 - 3-(Triethoxysilyl)-N-[3-(triethoxysilyl)propyl]-1-propanamin 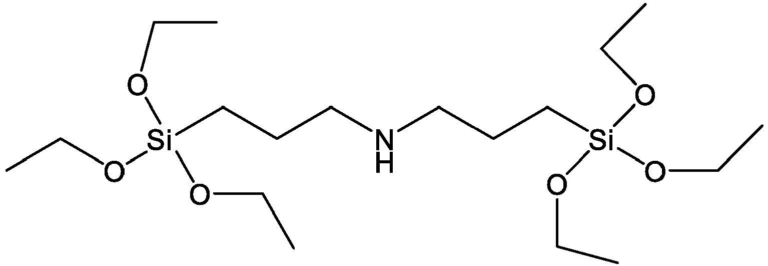 -N-Methyl-3-(trimethoxysilyl)-N-[3-(trimethoxysilyl)propyl]-1-propanamin 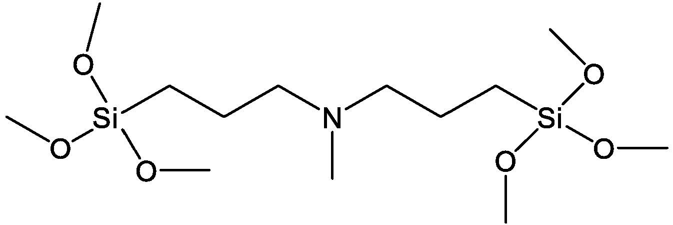 -N-Methyl-3-(triethoxysilyl)-N-[3-(triethoxysilyl)propyl]-1-propanamin 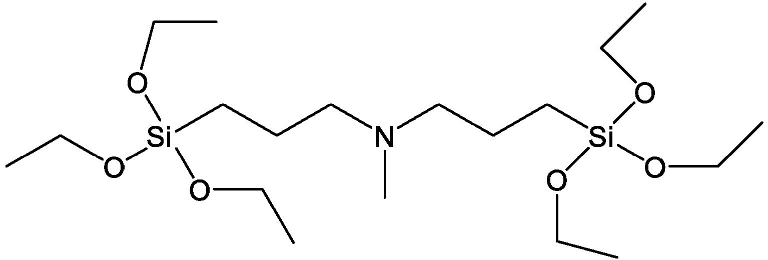 - 2-[Bis[3-(trimethoxysilyl)propyl]amino]-ethanol 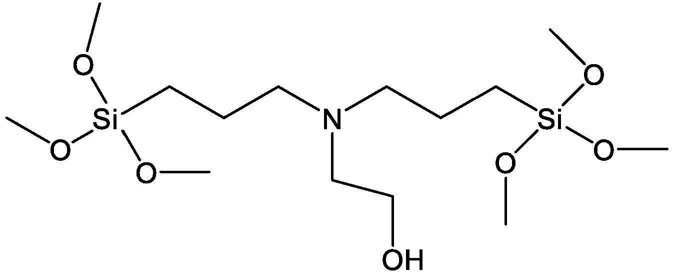 - 2-[Bis[3-(triethoxysilyl)propyl]amino]-ethanol 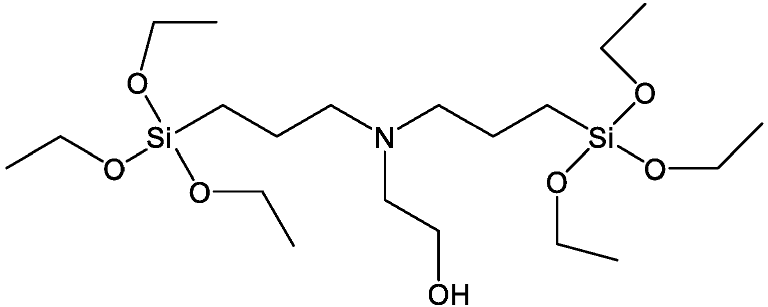 - 3-(Trimethoxysilyl)-N,N-bis[3-(trimethoxysilyl)propyl]-1-propanamin 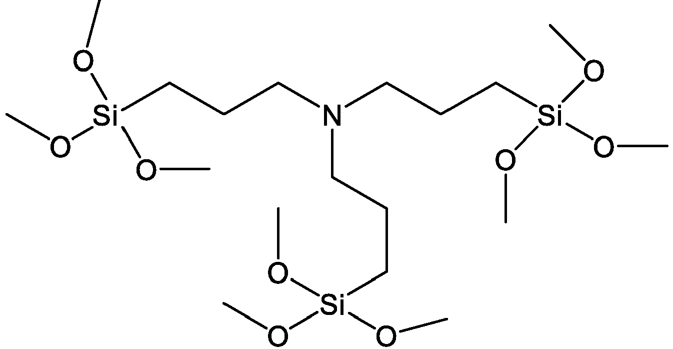 - 3-(Triethoxysilyl)-N,N-bis[3-(triethoxysilyl)propyl]-1-propanamin 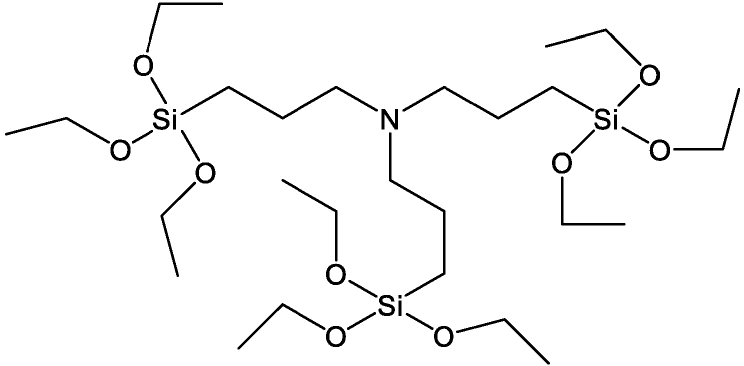 - N1,N1-Bis[3-(trimethoxysilyl)propyl]-1,2-ethanediamin, 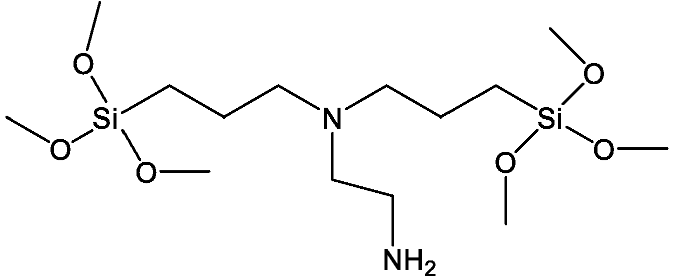 - N1,N1-Bis[3-(triethoxysilyl)propyl]-1,2-ethanediamin, 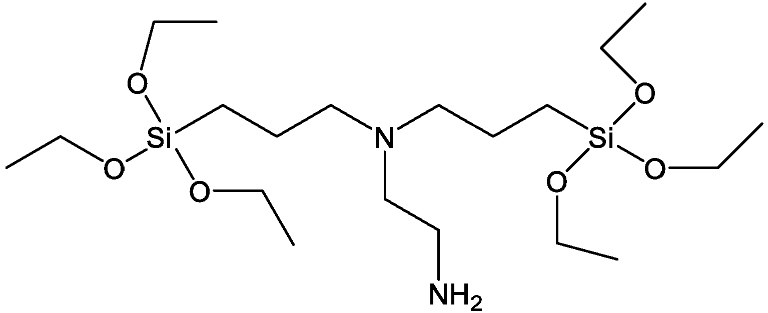 - N,N-Bis[3-(trimethoxysilyl)propyl]-2-propen-1-amin 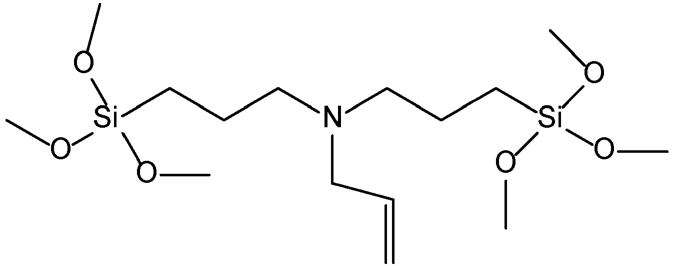 - N,N-Bis[3-(triethoxysilyl)propyl]-2-propen-1-amin

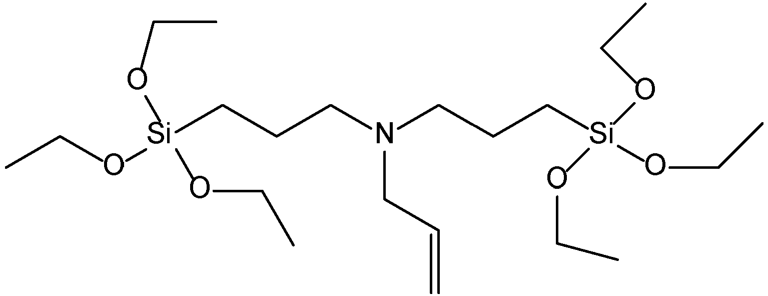

Die vorgenannten organische Siliciumverbindung der Formel (II) sind kommerziell erhältlich.

Bis(trimethoxysilylpropyl)amin mit der CAS-Nummer 82985-35-1 kann beispielsweise von Sigma-Aldrich käuflich erworben werden.

Bis[3-(triethoxysilyl)propyl]amin, auch bezeichnet als 3-(Triethoxysilyl)-N-[3-(triethoxysilyl)propyl]-1-propanamin, mit der CAS-Nummer 13497-18-2 kann zum Beispiel von Sigma-Aldrich käuflich erworben werden oder ist im Handel unter der Produktbezeichnung Dynasylan 1122 von Evonik erhältlich.

N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.

3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

Das auf dem Haar angewendete Mittel zur Behandlung eines keratinischen Materials enthält mindestens eine organische Siliciumverbindung der Formel (IV)

R₉Si(OR₁₀)ₖ(R₁₁)ₘ (IV).

Die Verbindungen der Formel (IV) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

Das bzw. die organischen Siliciumverbindungen der Formel (IV) können auch als Silane vom Typ der Alkylalkoxysilane oder der Alkylhydroxysilane bezeichnet werden,

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform enthält das Mittel zur Behandlung eines keratinischen Materials zusätzlich zu der oder den organischen Siliciumverbindungen der Formel (I) mindestens eine weitere organische Siliciumverbindung der Formel (IV)

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer ebenfalls bevorzugten Ausführungsform enthält das Mittel zur Behandlung eines keratinischen Materials zusätzlich zu der oder den organischen Siliciumverbindungen der Formel (II) mindestens eine weitere organische Siliciumverbindung der Formel (IV) enthält

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

Das Mittel zur Behandlung eines keratinischen Materials enthält zusätzlich zu den organischen Siliciumverbindungen der Formel (I) mindestens eine weitere organische Siliciumverbindung der Formel (IV)

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In den organischen Siliciumverbindungen der Formel (IV) steht der Rest R₉ für eine C₁-C₁₂-Alkylgruppe. Diese C₁-C₁₂-Alkylgruppe ist gesättigt und kann linear oder verzweigt sein. Bevorzugt steht R9 für eine lineare C₁-C₈-Alkylgruppe. Bevorzugt steht R₉ für eine Methylgruppe, eine Ethylgruppe, eine n-Propylgruppe, eine n-Butylgruppe, eine n-Pentylgruppe, eine n-Hexylgruppe, eine n-Octylgruppe oder eine n-Dodecylgruppe. Besonders bevorzugt steht R₉ für eine Methylgruppe, eine Ethylgruppe oder eine n-Octylgruppe.

In den organischen Siliciumverbindungen der Formel (IV) steht der Rest R₁₀ für ein Wasserstoffatom oder eine C₁-C₆-Alkylgruppe. Besonders bevorzugt steht R₁₀ für eine Methylgruppe oder für eine Ethylgruppe.

In den organischen Siliciumverbindungen der Formel (IV) steht der Rest R₁₁ für eine C₁-C₆-Alkylgruppe. Besonders bevorzugt steht R₁₁ für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Eine sehr hohe "Anti-Pollution"-Wirkung konnte erhalten werden, wenn das Mittel zur Behandlung eines keratinischen Materials mindestens eine organische Siliciumverbindung der Formel (IV) enthält, in welcher der Rest k für die Zahl 3 steht. In diesem Fall steht der Rest m für die Zahl 0.

Zur Lösung der Aufgabenstellung besonders gut geeignete organische Siliciumverbindungen der Formel (IV) sind
- Methyltrimethoxysilan 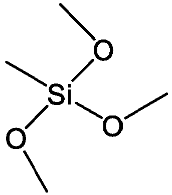
- Methyltriethoxysilan 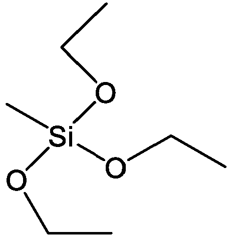
- Ethyltrimethoxysilan 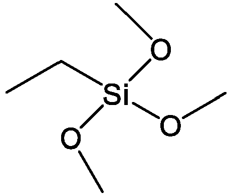
- Ethyltriethoxysilan 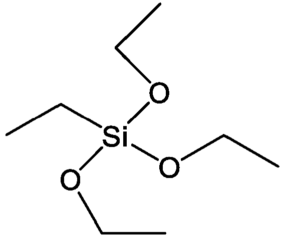
- n-Hexyltrimethoxysilan 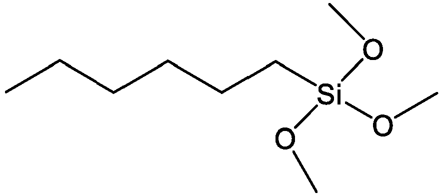
- n-Hexyltriethoxysilan 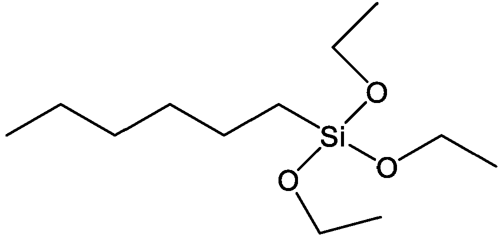
- n-Octyltrimethoxysilan 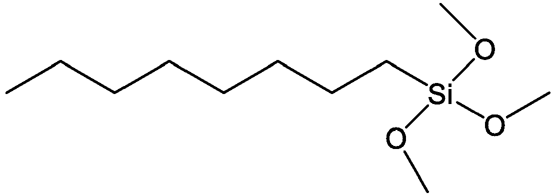
- n-Octyltriethoxysilan 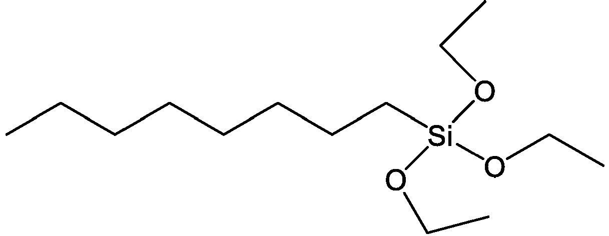
- n-Dodecyltrimethoxysilan und/oder 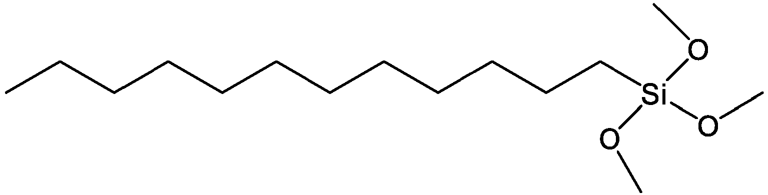
- n-Dodecyltriethoxysilan. 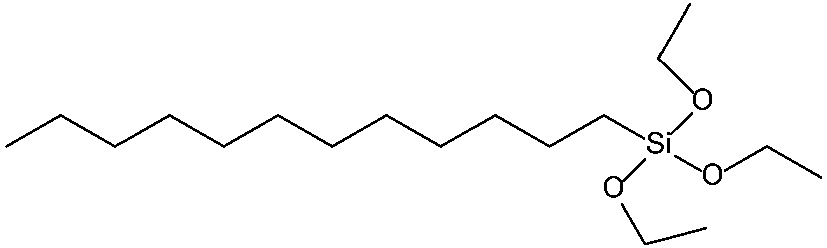 sowie Propyltrimethoxysilan, Propyltriethoxysilan, Octadecyltrimethoxysilan und/oder Octadecyltriethoxysilan.

Bei den zuvor beschriebenen organischen Siliciumverbindungen handelt es sich um reaktive Verbindungen.

Es hat sich herausgestellt, dass auf dem keratinischen Material auch dann besonders stabile und gleichmäßige Filme erhalten werden konnten, wenn das Mittel zwei strukturell voneinander verschiedene organische Siliciumverbindungen enthält.

Das Mittel ist dadurch gekennzeichnet, dass es mindestens eine organische Silicumverbindungen der Formel (I) und mindestens eine organische Silicumverbindung der Formel (IV) enthält.

In einer explizit ganz besonders bevorzugten Ausführungsform ist ein Mittel dadurch gekennzeichnet, dass es mindestens eine organische Silicumverbindungen der Formel (I) enthält, welche aus der Gruppe aus (3-Aminopropyl)triethoxysilan und (3-Aminopropyl)trimethoxysilan ausgewählt ist, und zusätzlich mindestens eine organische Silicumverbindungen der Formel (IV) enthält, welche aus der Gruppe bestehend aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan und Hexyltriethoxysilan ausgewählt ist.

In einer weiteren bevorzugten Ausführungsform ist ein Mittel dadurch gekennzeichnet, dass das Mittelbezogen auf das Gesamtgewicht des Mittels - enthält:
- 0,5 bis 5 Gew.-% mindestens einer ersten organischen Siliciumverbindung, die ausgewählt ist aus der Gruppe aus (3-Aminopropyl)trimethoxysilan, (3-Aminopropyl)triethoxysilan, (2-Aminoethyl)trimethoxysilan, (2-Aminoethyl)triethoxysilan, (3-Dimethylaminopropyl)trimethoxysilan, (3-Dimethylaminopropyl)triethoxysilan (2-Dimethylaminoethyl)trimethoxysilan und (2-Dimethylaminoethyl)triethoxysilan, und
- 3,2 bis 10 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octyldecyltrimethoxysilan und Octyldecyltriethoxysilan.

Bereits der Zusatz geringer Wassermengen führt bei organischen Siliciumverbindungen mit mindestens einer hydrolysierbaren Gruppe zur Hydrolyse. Die Hydrolyseprodukte und/oder organischen Siliciumverbindungen mit mindestens einer Hydroxygruppe können in einer Kondensationsreaktion miteinander reagieren. Aus diesem Grund können sowohl die siliciumorganischen Verbindungen mit mindestens einer hydrolysierbaren Gruppe als auch deren Hydrolyse- und/oder Kondensationsprodukte in dem Mittel enthalten sein. Bei Verwendung von siliciumorganischen Verbindungen mit mindestens einer Hydroxylgruppe können sowohl die organischen Siliciumverbindungen mit mindestens einer Hydroxylgruppe als auch deren Kondensationsprodukte in dem Mittel enthalten sein.

Unter einem Kondensationsprodukt wird ein Produkt verstanden, dass durch Reaktion von mindestens zwei organischen Siliciumverbindungen mit jeweils mindestens einer Hydroxylgruppen oder hydrolysierbaren Gruppen pro Molekül unter Abspaltung von Wasser und/oder unter Abspaltung von einem Alkanol entsteht. Die Kondensationsprodukte können beispielsweise Dimere, aber auch Trimere oder Oligomere sein, wobei die Kondensationsprodukte mit den Monomeren im Gleichgewicht stehen. Abhängig von der eingesetzten oder in der Hydrolyse verbrauchten Wassermenge verschiebt sich das Gleichgewicht von monomerer organischen Siliciumverbindungen zu Kondensationsprodukt.

Im Rahmen der vorliegenden Erfindung sind Angaben in Gew.-% - falls nicht anders angegeben - immer bezogen auf das Gesamtgewicht des kosmetischen Mittels.

Als zweiten erfindungswesentlichen Bestandteil enthält das kosmetische Mittel zur Behandlung eines keratinischen Materials mindestens ein Polyorganosiloxan, umfassend PCA (Pyrrolidoncarbonsäure) Dimethicone/Amodimethicone (INCI). Im Zuge der zu dieser Erfindung führenden Arbeiten hat sich herausgestellt, dass zur Erzielung einer besonders guten Pflege-Wirkung es insbesondere von Vorteil ist, wenn die organischen Siliciumverbindungen mit einem Polyorganosiloxan kombiniert werden. Das Polyorganosiloxan unterscheidet sich von der organischen Siliciumverbindung.

Unter dem Begriff "Polyorganosiloxan" ist ein Polymer zu verstehen, dessen Polymerrückgrates eine Si-O-Kette aufweist, d.h. bei denen Siliciumatome über ein Sauerstoffatom im Polymerrückgrat verbunden ist.

Die Kombination aus der organischen Siliciumverbindung mit einem Polyorganosiloxan bildet eine Schicht auf dem Haar. Dadurch wird gewährleistet, dass oxidative Haarfarben signifikant vor dem Auswaschen geschützt werden. Ferner wird die Haaroberfläche bei oxidativ geschädigtem Haar wieder hydrophobisiert, was zur Reduktion von Frizz führt. Darüber hinaus wird die Kämmbarkeit des Haares verbessert.

Das Polysiloxan ist DIMETHYLAMINOPROPYLAMIDO PCA DIMETHICONE (INCI), das auch als PCA (Pyrrolidoncarbonsäure) Dimethicone/Amodimethicone (INCI) bezeichnet wird. Dieses umfasst Polydimethylsiloxan-Einheiten und eine Siloxan-Einheit, die eine über eine Propylengruppe gebundene Pyrrolidoncarbonsäure umfasst, wie in der folgenden Formel dargestellt ist:

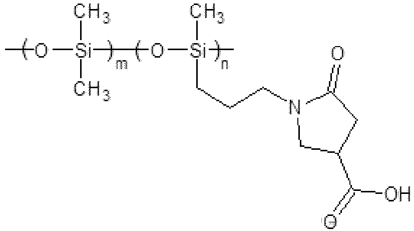

In weiteren bevorzugten Ausführungsformen können die folgenden Siloxangruppen enthaltenden Polymere zusätzlich zu den zwingend enthaltenen Komponenten a) und b) in einem erfindungsgemäßen Mittel enthalten: Acrylates/Octylacrylamide/Diphenyl Amodimethicone Copolymer (INCI), Bis-Butyloxyamodimethicone/PEG-60 Copolymer (INCI), Butyl Acrylate/Hydroxypropyl Dimethicone Acrylate Copolymer (INCI), Isobutylmethacrylate/Bis-Hydroxypropyl Dimethicone Acrylate Copolymer (INCI), PEG/PPG-25/25 Dimethicone/Acrylates Copolymer (INCI), Dimethicone Crosspolymer (INCI), Diphenyl Amodimethicone (INCI).

Gemäß einer bevorzugten Ausführungsform der vorliegenden Erfindung beträgt die Menge an Polyorganosiloxan in dem kosmetischen Mittel von 0,1 bis 30 Gew.-%, bevorzugt von 0,5 bis 20 Gew.-%, bevorzugter von 1 bis 12 Gew.-%, noch bevorzugter 1,5 bis 9 Gew.-%, am meisten bevorzugt 2 bis 6 Gew.-%, bezogen auf das Gesamtgewicht des kosmetischen Mittels.

Das kosmetische Mittel zur Behandlung eines keratinischen Materials kann insbesondere ein Mittel zur Reinigung eines keratinischen Materials, ein Mittel zur Pflege eines keratinischen Materials und ein Mittel zur Pflege und Reinigung eines keratinischen Materials umfassen.

Im Folgenden werden weitere Bestandteile der Haarbehandlungsmittel beschrieben, die neben den zuvor beschriebenen zwingenden Komponenten in den Mitteln enthalten sein können.

Es kann bevorzugt sein, dass das Mittel zur Behandlung eines keratinischen Materials ferner 0,001 bis 20 Gew.-% mindestens einer quaternären Verbindung umfasst. Dies gilt insbesondere für Mittel zur Pflege eines keratinischen Materials und für Mittel zur Pflege und Reinigung eines keratinischen Materials.

Es ist bevorzugt, dass die mindestens eine quaternäre Verbindung ausgewählt ist aus mindestens einer der Gruppen bestehend aus
i) der Monoalkylquats und/oder
ii) der Esterquats und/oder
iii) der quaternären Imidazoline der Formel (Tkat2), 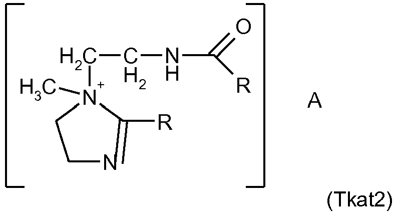 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
iv) Polyquaternium-37, und/oder
v) Poly(methacryloyloxyethyltrimethylammoniumverbindungen) und/oder;
vi) quaternisierten Cellulose-Derivaten, insbesondere Polyquaternium 10, Polyquaternium-24, Polyquaternium-27, Polyquaternium-67, Polyquaternium-72, und/oder
vii) kationischen Alkylpolyglycosiden und/oder
viii) kationisiertem Honig und/oder
ix) kationischen Guar-Derivaten und/oder
x) Chitosan und/oder
xi) polymeren Dimethyldiallylammoniumsalzen und deren Copolymeren mit Estern und Amiden von Acrylsäure und Methacrylsäure, insbesondere Polyquaternium-7 und/oder
xii) Copolymeren des Vinylpyrrolidons mit quaternierten Derivaten des Dialkylaminoalkylacrylats und -methacrylats, insbesondere Polyquaternium-11 und/oder
xiii) Vinylpyrrolidon-Vinylimidazoliummethochlorid-Copolymeren, insbesondere Polyquaternium-16 und/oder
xiv) quaterniertem Polyvinylalkohol und/oder
xv) Polyquaternium-74,
sowie Mischungen hiervon. Es ist insbesondere bevorzugt, dass das Haarbehandlungsmittel ein kationisches Hompolymer, welches unter die INCI-Bezeichnung Polyquaternium-37 fällt, als quaternäre Verbindungen enthält.

In dem kosmetischen Mittel können kationische Tenside enthalten sein. Ein kationisches Tensid umfasst eine hydrophobe Kopfgruppe mit einer kationischen Ladung und einen oder zwei hydrophobe Endteile, wobei das hydrophobe Endteil oder die hydrophoben Endteile geradkettige oder verzweigte, gesättigte oder ein- oder mehrfach ungesättigte Alkylgruppen darstellen, die bevorzugt eine Kettenlänge von C6 bis C30, bevorzugter C8 bis C26, besonders bevorzugt C10 bis C22, aufweisen. Gemäß einer weiteren bevorzugten Ausführungsform weist das kationische Tensid eine Esterfunktion, eine Etherfunktion, eine Ketonfunktion, eine Alkoholfunktion oder eine Amidfunktion auf.

Das kosmetische Mittel kann ein kationisches Tensid der Formel (V) enthalten, 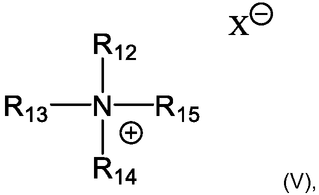 worin
- R₁₂, R₁₃, R₁₄: unabhängig voneinander für eine C1-C6-Alkylgruppe, eine C2-C6-Alkenylgruppe oder eine C2-C6-Hydroxyalkylgruppe stehen,
- R₁₅: für eine C8-C28-Alkylgruppe, bevorzugt eine C10-C22-Alkylgruppe steht und
- X-: für ein physiologisch verträgliches Anion steht,
und/oder das kosmetische Mittel kann mindestens ein kationisches Tensid der Formel (VI) enthalten, 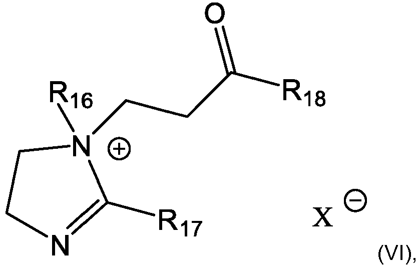 worin
- R₁₆: für eine C1-C6-Alkylgruppe steht
- R₁₇, R₁₈: unabhängig voneinander für eine C7-C27-Alkylgruppe, bevorzugt eine C10-C22-Alkylgruppe stehen und
- X-: für ein physiologisch verträgliches Anion steht,
und/oder das kosmetische Mittel kann mindestens ein kationisches Tensid der Formel (VII) enthalten, 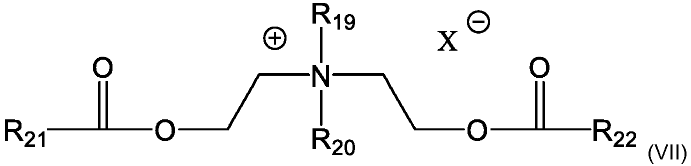 worin
- R₁₉, R₂₀: unabhängig voneinander für eine C1-C6-Alkylgruppe oder eine C2-C6-Hydroxyalkylgruppe stehen,
- R₂₁, R₂₂: unabhängig voneinander für eine C7-C27-Alkylgruppe, bevorzugt eine C10-C22-Alkylgruppe stehen und
- X-: für ein physiologisch verträgliches Anion steht.

Ferner können die kosmetischen Mittel gemäß der vorliegenden Erfindung anionische Tenside enthalten. Bevorzugte anionische Tenside sind ausgewählt aus der Gruppe bestehend aus
- geradkettigen oder verzweigten, gesättigten oder ein- oder mehrfach ungesättigten Alkylsulfonaten mit 8 bis 24, bevorzugt 12 bis 22, bevorzugter 16 bis 18 C-Atomen,
- linearen Alpha-Olefinsulfonaten mit 8 bis 24, bevorzugt 12 bis 22, bevorzugter 16 bis 18 C-Atomen,
- Alkylsulfaten und Alkylpolyglykolethersulfaten der Formel R₉-O-(CH₂-CH₂O)ₙ-SO₃X, in der R₉ bevorzugt für einen geradkettigen oder verzweigten, gesättigten oder ein- oder mehrfach ungesättigten Alkyl- oder Alkenlyrest mit 8 bis 24, bevorzugt 12 bis 22, bevorzugter 16 bis 18 Kohlenstoffatomen, n für 0 oder 1 bis 12, bevorzugter 2 bis 4 und X für ein Alkali- oder Erdalkalimetallion oder für protoniertes Triethanolamin oder das Ammonium-Ion steht,
- geradkettigen oder verzweigten, gesättigten oder ein- oder mehrfach ungesättigten Alkylcarbonsäuren mit 8 bis 24, bevorzugt 12 bis 22, bevorzugter 16 bis 18 C-Atomen,
- geradkettigen oder verzweigten, gesättigten oder ein- oder mehrfach ungesättigten Alkylphosphaten mit 8 bis 24, bevorzugt 12 bis 22, bevorzugter 16 bis 18 C-Atomen,
- Alkylisethionat, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe, insbesondere Natriumcocoylisethionat,
- Alkylglycosidcarbonsäuren, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Alkylsulfosuccinaten, dessen zwei Alkylgruppen ausgewählt sind aus gleichen oder verschiedenen, verzweigte oder unverzweigten C₂ bis C₁₂, bevorzugt C₄ bis C₁₀, bevorzugter C₆ bis C₈ Alkylgruppen,
- Alkyltauraten, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Alkylsarcosinaten, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Sulfonate ungesättigter Fettsäuren mit 8 bis 24, bevorzugt 12 bis 22, bevorzugter 16 bis 18 C-Atomen und 1 bis 6 Doppelbindungen,
wobei das Gegenion des anionischen Tensids ein Alkali- oder Erdalkalimetallion oder ein protoniertes Triethanolamin oder das Ammonium-Ion ist.

Besonders bevorzugte anionische Tenside sind geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten enthalten. Ganz besonders bevorzugt enthält die Tensidmischung aus anionischen und amphoteren/zwitterionischen Tensiden Natriumlaurylethersulfat (INCI: Sodium Laureth Sulfate) und ganz besonders bevorzugt Natriumlaurylethersulfat mit 2 Ethylenoxideinheiten.

Ferner können die kosmetischen Mittel gemäß der vorliegenden Erfindung amphotere Tenside enthalten. Amphotere Tenside, welche auch als zwitterionische Tenside bezeichnet werden, werden solche oberflächenaktiven Verbindungen genannt, die im Molekül mindestens eine quartäre Ammoniumgruppe und mindestens eine -COO⁻ - oder -S0₃⁻ -Gruppe tragen. Unter amphoteren/zwitterionischen Tensiden werden auch solche oberflächenaktiven Verbindungen verstanden, die außer einer C₈ - C₂₄-Alkyl- oder - Acylgruppe mindestens eine freie Aminogruppe und mindestens eine -COOH- oder -SO₃H-Gruppe enthalten und zur Ausbildung innerer Salze befähigt sind.

Bevorzugte amphotere Tenside in den kosmetischen Mitteln sind ausgewählt aus der Gruppe bestehend aus
- Alkylbetain, umfassend mindestens eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylamphodiacetat oder Alkylamphodiacetat, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe, mit einem Alkali- oder Erdalkalimetallgegenion, und
- Alkylamidopropylbetain, umfassend mindestens eine gesättigte oder ungesättigte, verzweigte oder unverzweigte C₆ bis C₂₂, bevorzugt C₁₀ bis C₁₈, bevorzugter C₁₂ bis C₁₆ Alkylgruppe.

Zu den insbesondere geeigneten amphoteren/zwitterionischen Tensiden zählen die unter der INCI-Bezeichnung bekannten Tenside Cocamidopropylbetain und Disodium Cocoamphodiacetate.

Gemäß einer bevorzugten Ausführungsform der vorliegenden Erfindung ist das nichtionische Tensid ausgewählt aus der Gruppe bestehend aus
- Alkylglucamid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylfructosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylglucosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylalkoholalkoxylat der Formel R₁₀(OR₁₁)ₘOH, in der R₁₀ eine lineare oder verzweigte C₆ bis C₂₂, bevorzugt C₁₀ bis C₁₈, bevorzugter C₁₂ bis C₁₆ Alkylgruppe, R₁₁ eine C₂ bis C₄, bevorzugt eine C₂ Alkylgruppe, und m 1 bis 10, bevorzugt 2 bis 6, bevorzugter 2 bis 6, darstellen, und
- Alkylester der Formel R₁₂COOR₁₃, in der R₁₂ eine lineare oder verzweigte C₆ bis C₂₂, bevorzugt C₁₀ bis C₁₈, bevorzugter C₁₂ bis C₁₆ Alkylgruppe, R₁₃ eine C₁ bis C₄, bevorzugt eine C₂ Alkylgruppe, darstellen.

Es kann bevorzugt sein, dass das Mittel zur Behandlung eines keratinischen Materials ferner eine festigende Verbindung, vorzugsweise ausgewählt aus der Gruppe bestehend aus Wachsen, synthetischen Polymeren und Mischungen daraus, umfasst.

Die synthetischen Polymere lassen sich in kationische, anionische, nichtionische und amphotere Polymere unterteilen.

Geeignete synthetische Polymere umfassen beispielsweise Polymere mit den folgenden INCI-Bezeichnungen: Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Stearyl Acrylate/Ethylamine Oxide Methacrylate Copolymer, Acrylates/VA Copolymer, Acrylates/Hydroxyesters Acrylates Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Butyl Acrylate/Ethylhexyl Methacrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP/VA/ltaconic Acid Copolymer, PVP/VA/Vinyl Propionate Copolymer, Polyvinylpyrrolidon (PVP), Vinylpyrrolidon-Vinylacetat-Copolymer (VP/VA Coplymer), Vinyl Caprolactam/VP/Dimethylaminoethyl Methacrylate Copolymer (INCI), VP/DMAPA Acrylates Copolymer (INCI), Octylacrylamide/Acrylates/Butylaminoethyl Methacrylate Copolymer (INCI), Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer. Ebenso geeinet sind Celluloseether, wie Hydroxypropylcellulose, Hydroxyethylcellulose und Methylhydroxypropylcellulose.

Die kosmetische Zusammensetzung kann zusätzlich oder alternativ zu einem synthetischen Polymer mindestens ein natürliches oder synthetisches Wachs, welches einen Schmelzpunkt von über 37 °C aufweist, als festigende Verbindung enthalten.

Als natürliche oder synthetische Wachse können feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie zum Beispiel Bienenwachse und andere Insektenwachse, Walrat, Schellackwachs, Wollwachs und Bürzelfett, weiterhin Mineralwachse, wie zum Beispiel Ceresin und Ozokerit oder die petrochemischen Wachse, wie zum Beispiel Petrolatum, Paraffinwachse, Microwachse aus Polyethylen oder Polypropylen und Polyethylenglycolwachse eingesetzt werden. Es kann vorteilhaft sein, hydrierte oder gehärtete Wachse einzusetzen. Weiterhin sind auch chemisch modifizierte Wachse, insbesondere die Hartwachse, zum Beispiel Montanesterwachse, Sasolwachse und hydrierte Jojobawachse, einsetzbar.

Weiterhin geeignet sind die Triglyceride gesättigter und gegebenenfalls hydroxylierter C16-30-Fettsäuren, wie zum Beispiel gehärtete Triglyceridfette (hydriertes Palmöl, hydriertes Kokosöl, hydriertes Rizinusöl), Glyceryltribehenat oder Glyceryltri-12-hydroxystearat.

Die Wachskomponenten können auch aus der Gruppe der Ester aus gesättigten, unverzweigten Alkancarbonsäuren einer Kettenlänge von 22 bis 44 C-Atomen und gesättigten, unverzweigten Alkoholen einer Kettenlänge von 22 bis 44 C-Atomen ausgewählt werden, sofern die Wachskomponente oder die Gesamtheit der Wachskomponenten bei Raumtemperatur fest sind. Auch Silikonwachse, zum Beispiel Stearyltrimethylsilan/Stearylalkohol sind gegebenenfalls vorteilhaft, sollen aber nicht zu den organischen Siliciumverbindungen (Komponente a) gezählt werden.

Natürliche, chemisch modifizierte und synthetische Wachse können alleine oder in Kombination eingesetzt werden. Es können somit auch mehrere Wachse eingesetzt werden. Weiterhin ist auch eine Reihe von Wachsmischungen, ggf. in Abmischung mit weiteren Zusätzen, im Handel erhältlich. Die unter den Bezeichnungen "Spezialwachs 7686 OE" (eine Mischung aus Cetylpalmitat, Bienenwachs, mikrokristallinem Wachs und Polyethylen mit einem Schmelzbereich von 73-75 °C; Hersteller: Kahl & Co), Polywax^{®} GP 200 (eine Mischung von Stearylalkohol und Polyethylenglykolstearat mit einem Schmelzpunkt von 47-51 °C; Hersteller: Croda) und "Weichceresin^{®} FL 400" (ein Vaseline/Vaselinöl/Wachs-Gemisch mit einem Schmelzpunkt von 50-54 °C; Hersteller: Parafluid Mineralölgesellschaft) sind Beispiele für einsetzbare Mischungen.

Bevorzugt ist das Wachs ausgewählt aus Carnaubawachs (INCI: Copernicia Cerifera Cera) Bienenwachs (INCI: Beeswax), Petrolatum (INCI), mikrokristallinem Wachs und insbesondere Gemischen daraus.

Bevorzugte Mischungen umfassen die Kombination von Carnaubawachs (INCI: Copernicia Cerifera Cera), Petrolatum und mikrokristallinem Wachs oder die Kombination von Bienenwachs (INCI: Beeswax) und Petrolatum.

Das Wachs oder die Wachskomponenten sollten bei 25 °C fest sein und sollen im Bereich von > 37 °C schmelzen.

Weitere geeignete Inhaltsstoffe umfassen nichtionische Polymere, anionische Polymere, (weitere) kationische Polymere, Wachse, Proteinhydrolysate, Aminosäuren, Oligopetide, Vitamine, Provitamine, Vitaminvorstufen, Betaine, Biochinone, Purin(derivate), Pflegestoffe, Pflanzenextrakte, Esteröle, UV-Lichtschutzfilter, Strukturierungsmittel, Verdickungsmittel, Elektrolyte, pH-Stellmittel, Quellmittel, Farbstoffe, Antischuppenwirkstoffe, Komplexbildner, Trübungsmittel, Perlglanzmittel, Pigmente, Stabilisierungsmittel, Treibmittel, Antioxidantien, Parfümöle und/oder Konservierungsmittel.

In den bevorzugten Ausführungsformen 1 bis 14 werden die bevorzugten organischen Siliciumverbindungen a) mit dem erfindungsgemäßen Polyorganosiloxan b) kombiniert. Die Kombinationen der aufgeführten Komponenten a) und b) werden mit den weiteren Inhaltsstoffen, die gemäß der vorliegenden Erfindung verwendet werden können, zu bevorzugten kosmetischen Mitteln kombiniert.

| | Silanverbindung | weiterer Inhaltsstoff |
|---|---|---|
| 1 | (3-Aminopropyl)triethoxysilan + Methyltrimethoxysilan | PCA (Pyrrolidoncarbonsäure) Dimethicone/Amodimethicone (INCI) PCA ( |
| 2 | (3-Aminopropyl)triethoxysilan + Methyltriethoxysilan | Pyrrolidoncarbonsäure) Dimethicone/Amodimethicone (INCI) |
| 3 | (3-Aminopropyl)triethoxysilan + Ethyltrimethoxysilan | PCA (Pyrrolidoncarbonsäure) Dimethicone/Amodimethicone (INCI) |
| 4 | (3-Aminopropyl)triethoxysilan + Ethyltriethoxysilan | PCA (Pyrrolidoncarbonsäure) Dimethicone/Amodimethicone (INCI) |
| 5 | (3-Aminopropyl)triethoxysilan + Propyltrimethoxysilan | PCA (Pyrrolidoncarbonsäure) Dimethicone/Amodimethicone (INCI) |
| 6 | (3-Aminopropyl)triethoxysilan + Propyltriethoxysilan | PCA (Pyrrolidoncarbonsäure) Dimethicone/Amodimethicone (INCI) |
| 7 | (3-Aminopropyl)triethoxysilan + Hexyltrimethoxysilan | PCA (Pyrrolidoncarbonsäure) Dimethicone/Amodimethicone (INCI) PCA ( |
| 8 | (3-Aminopropyl)triethoxysilan + Hexyltriethoxysilan | Pyrrolidoncarbonsäure) Dimethicone/Amodimethicone (INCI) |
| 9 | (3-Aminopropyl)triethoxysilan + Octyltrimethoxysilan | PCA (Pyrrolidoncarbonsäure) Dimethicone/Amodimethicone (INCI) |
| 10 | (3-Aminopropyl)triethoxysilan + Octyltriethoxysilan | PCA (Pyrrolidoncarbonsäure) Dimethicone/Amodimethicone (INCI) |
| 11 | (3-Aminopropyl)triethoxysilan + Dodecyltrimethoxysilan | PCA (Pyrrolidoncarbonsäure) Dimethicone/Amodimethicone (INCI) |
| 12 | (3-Aminopropyl)triethoxysilan + Dodecyltriethoxysilan | PCA (Pyrrolidoncarbonsäure) Dimethicone/Amodimethicone (INCI) |
| 13 | (3-Aminopropyl)triethoxysilan + Octadecyltrimethoxysilan | PCA (Pyrrolidoncarbonsäure) Dimethicone/Amodimethicone (INCI) PCA ( |
| 14 | (3-Aminopropyl)triethoxysilan + Octadecyltriethoxysilan | Pyrrolidoncarbonsäure) Dimethicone/Amodimethicone (INCI) |

Die Wirkstoffkombination aus mindestens einer organischen Siliciumverbindung und einem Polyorganosiloxan kann bereits im Mittel zur Behandlung eines keratinischen Materials enthalten sein. In dieser Ausführungsform wird das Mittel zur Behandlung eines keratinischen Materials bereits in anwendungsbereiter Form vertrieben.

Alternativ wird die mindestens eine organische Siliciumverbindung einer Minute bis 12 Stunden, bevorzugt 5 Minuten bis 6 Stunden, mehr bevorzugt 10 Minuten bis 3 Stunden, noch mehr bevorzugt 30 Minuten bis einer Stunde vor Anwendung des Mittels zur Behandlung eines keratinischen Materials einer Basis umfassend alle Inhaltsstoffe des Mittels zur Behandlung eines keratinischen Materials mit Ausnahme der mindestens einen organischen Siliciumverbindung zugefügt.

Ferner werden alternativ die organische Siliciumverbindung und das Polyorganosiloxan einem kosmetischen Produkt erst kurz vor der Anwendung, d.h. 1 Minute bis 12 Stunden, bevorzugt 2 Minuten bis 6 Stunden, besonders bevorzugt 1 Minute bis 3 Stunden, insbesondere bevorzugt 1 Minute bis 1 Stunde, zugegeben.

In einer weiteren Alternative wird die organische Siliciumverbindung einer wässrigen Lösung zugegeben, welche auf das Haar appliziert wird, und im zweiten Schritt wird eine wässrige Lösung oder ein kosmetisches Mittel, welches das Polyorganosiloxan enthält, auf das Haar aufgetragen.

Beispielsweise kann der Anwender ein Mittel (α), welches die organische Siliciumverbindung(en) enthält, zunächst mit einem Mittel (β), welches die restlichen Inhaltsstoffe des Mittels zur Behandlung eines keratinischen Materials umfasst, verrühren oder verschütteln. Diese Mischung aus (α) und (β) kann der Anwender nun - entweder direkt nach ihrer Herstellung oder nach einer kurzen Reaktionszeit von 1 Minute bis 20 Minuten - auf die keratinischen Materialien applizieren. Das Mittel (β) kann Wasser enthalten, insbesondere Wasser in einer Menge > 30 Gew.-%, bezogen auf das Gesamtgewicht des Mittels zur Behandlung keratinischer Materialien.

Ein weiterer Gegenstand der vorliegenden Anmeldung ist die Verwendung eines erfindungsgemäßen kosmetischen Mittels zur Behandlung eines keratinischen Materials
zur Pflege von keratinischem Material,
zur Reduzierung und/oder Verhinderung von schädlichen Auswirkungen von Luft- und Wasserverunreinigungen auf keratinisches Material und/oder
zur Reduzierung und/oder Verhinderung des Verblassens von oxidativ gefärbtem keratinischen Materials.

Bezüglich weiterer bevorzugter Ausführungsformen der Verwendung gilt mutatis mutandis das zu den kosmetischen Mitteln Gesagte.

## Patentansprüche

1. Kosmetisches Mittel zur Behandlung eines keratinischen Materials, umfassend
a) mindestens eine organische Siliciumverbindung, die mindestens eine organische Siliciumverbindung eine Verbindung der Formel (I) und mindestens eine organische Siliciumverbindung der Formel (IV) enthält, wobei in der organischen Siliciumverbindung der Formel (I)
R₁R₂N-L-Si(OR₃)a(R₄)b (I),
- R₁, R₂ beide für ein Wasserstoffatom stehen,
- L für eine lineare, zweibindige C₁-C₆-Alkylengruppe steht,
- R₃, R₄ unabhängig voneinander für eine Methylgruppe oder für eine Ethylgruppe stehen,
- a für die Zahl 3 steht und
- b für die Zahl 0 steht, und
wobei in der organischen Siliciumverbindung der Formel (IV)
R₉Si(OR₁₀)ₖ(R₁₁)ₘ (IV),
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht, und
b) mindestens ein Polyorganosiloxan, umfassend PCA (Pyrrolidoncarbonsäure) Dimethicone/Amodimethicone (INCI).

2. Kosmetisches Mittel zur Behandlung eines keratinischen Materials gemäß Anspruch 1,
**dadurch gekennzeichnet,**
**dass** die mindestens eine organische Siliciumverbindung ferner eine Verbindung der Formel (II) enthält,
wobei in der organischen Siliciumverbindung der Formel (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR_{5'})_{c} (II),
- R₅, R_{5'}, R_{5"}, R₆, R_{6'} und R_{6"} unabhängig voneinander für eine C₁-C₆-Alkylgruppe stehen,
- A, A', A", A‴ und Aʺʺ unabhängig voneinander für eine lineare oder verzweigte, zweibindige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-Alkylgruppe oder eine Gruppierung der Formel (III) stehen
- (Aʺʺ)-Si(R₆")_{d"}(OR₅")_{c"} (III),
- c für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
mit der Maßgabe, dass mindestens einer der Reste aus e, f, g und h von 0 verschieden ist.

3. Kosmetisches Mittel zur Behandlung eines keratinischen Materials gemäß einem der Ansprüche 1 oder 2,
**dadurch gekennzeichnet,**
**dass** das Mittel zur Behandlung eines keratinischen Materials mindestens eine organische Siliciumverbindung der Formel (I) enthält, die ausgewählt ist aus der Gruppe bestehend aus
- (3-Aminopropyl)trimethoxysilan
- (3-Aminopropyl)triethoxysilan
- (2-Aminoethyl)trimethoxysilan und
- (2-Aminoethyl)triethoxysilan.

4. Kosmetisches Mittel zur Behandlung eines keratinischen Materials gemäß einem der Ansprüche 1 bis 3, **dadurch gekennzeichnet, dass** die organische Siliciumverbindung der Formel (I) in einer Menge von 0,01 bis 10 Gew.-%, bevorzugt von 0,02 bis 8 Gew.-%, bevorzugter von 0,05 bis 6 Gew.-%, am meisten bevorzugt von 0,1 bis 4 Gew.-%, bezogen auf das Gesamtgewicht des kosmetischen Mittels, in dem kosmetischen Mittel enthalten ist.

5. Kosmetisches Mittel zur Behandlung eines keratinischen Materials gemäß einem der Ansprüche 1 bis 4, **dadurch gekennzeichnet, dass** das Polyorganosiloxan in einer Menge von 0,1 bis 30 Gew.-%, bevorzugt von 0,5 bis 20 Gew.-%, bevorzugter von 1 bis 12 Gew.-%, noch bevorzugter 1,5 bis 9 Gew.-%, am meisten bevorzugt 2 bis 6 Gew.-%, bezogen auf das Gesamtgewicht des kosmetischen Mittels, in dem kosmetischen Mittel enthalten ist.

6. Kosmetisches Mittel zur Behandlung eines keratinischen Materials gemäß einem der Ansprüche 1 bis 5, **dadurch gekennzeichnet, dass** das Mittel zur Behandlung eines keratinischen Materials mindestens eine organische Siliciumverbindung der Formel (IV) enthält,
R₉Si(OR₁₀)ₖ(R₁₁)ₘ (IV),
die ausgewählt ist aus der Gruppe bestehend aus
- Methyltrimethoxysilan
- Methyltriethoxysilan
- Ethyltrimethoxysilan
- Ethyltriethoxysilan
- Propyltriethoxysilan
- Propyltriethoxysilan
- Hexyltriethoxysilan
- Hexyltriethoxysilan
- Octyltrimethoxysilan
- Octyltriethoxysilan
- Dodecyltrimethoxysilan und
- Dodecyltriethoxysilan
- Octadecyltriethoxysilan und
- Octadecyltriethoxysilan.

7. Kosmetisches Mittel zur Behandlung eines keratinischen Materials gemäß einem der Ansprüche 1 bis 6, **dadurch gekennzeichnet, dass** das Mittel zur Behandlung eines keratinischen Materials- bezogen auf das Gesamtgewicht des Mittels zur Behandlung eines keratinischen Materials - enthält:
- 0,5 bis 3 Gew.-% mindestens einer ersten organischen Siliciumverbindung, die ausgewählt ist aus der Gruppe aus (3-Aminopropyl)trimethoxysilan, (3-Aminopropyl)triethoxysilan, (2-Aminoethyl)trimethoxysilan und (2-Aminoethyl)triethoxysilan und
- 3,2 bis 7 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan.

8. Verwendung eines kosmetischen Mittels zur Behandlung eines keratinischen Materials gemäß einem der Ansprüche 1 bis 7
zur Pflege von keratinischem Material,
zur Reduzierung und/oder Verhinderung von schädlichen Auswirkungen von Luft- und Wasserverunreinigungen auf keratinisches Material und/oder
zur Reduzierung und/oder Verhinderung des Verblassens von oxidativ gefärbtem keratinischen Materials.

## Claims

1. A cosmetic composition for treating a keratinous material, comprising
a) at least one organic silicon compound, wherein the organic silicon compound comprises at least one organic silicon compound of formula (I) and at least one organic silicon compound of formula (IV), wherein in the organic silicon compound of formula (I)
R₁R₂N-L-Si(OR₃)a(R₄)b (I),
- R₁, R₂ both represent a hydrogen atom,
- L represents a linear, divalent C₁-C₆ -alkylene group,
- R₃ and R₄ independently represent a methyl group or an ethyl group,
- a represents the number 3, and
- b represents the number 0, and
wherein, in the organic silicon compound of formula (IV)
R₉Si(OR₁₀)ₖ(R₁₁)ₘ (IV),
- R₉ represents a C₁-C₁₂ -alkyl group,
- R₁₀ represents a hydrogen atom or a C₁-C₆ -alkyl group,
- R₁₁represents a C₁-C₆alkyl group
- k represents an integer from 1 to 3, and
- m represents the integer 3 - k, and
b) at least one polyorganosiloxane comprising PCA (pyrrolidone carboxylic acid) dimethicone/amodimethicone (INCI).

2. A cosmetic composition for treating a keratinous material according to claim 1,
**characterized in**
**that** the at least one organic silicon compound further contains a compound of formula (II),
wherein, in the organic silicon compound of formula (II)
(R₅O)_{c}(R₆)_{d}Si-(A)ₑ-[NR₇-(A")]_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} and R_{6"} independently represent a C₁-C₆-alkyl group,
- A, A', A", A"', and Aʺʺ independently represent a linear or branched, divalent C₁-C₂₀-alkylene group,
- R₇ and R₈ independently represent a hydrogen atom, a C₁-C₆ -alkyl group, a hydroxy-C₁-C₆-alkyl group, a C₂-C₆alkenyl group, an amino-C₁-C₆-alkyl group, or a group of formula (III)
- (Aʺʺ)-Si(R₆")_{d"}(OR₅")_{c"} (III),
- c represents an integer from 1 to 3,
- d represents the integer 3 - c,
- c' represents an integer from 1 to 3,
- d' stands for the integer 3 - c',
- c" represents an integer from 1 to 3,
- d" stands for the integer 3 - c",
- e stands for 0 or 1,
- f stands for 0 or 1,
- g stands for 0 or 1,
- h is 0 or 1,
provided that at least one of the radicals e, f, g, and h is not 0.

3. A cosmetic composition for treating a keratinous material according to one of claims 1 or 2, **characterized in**
**that** the composition for treating a keratinous material contains at least one organic silicon compound of formula (I) selected from the group consisting of
- (3-aminopropyl)trimethoxysilane
- (3-aminopropyl)triethoxysilane
- (2-aminoethyl)trimethoxysilane, and
- (2-aminoethyl)triethoxysilane.

4. A cosmetic composition for treating a keratinous material according to any one of claims 1 to 3, **characterized in that** the organic silicon compound of formula (I) is contained in the cosmetic composition in an amount of 0.01 to 10 wt.%, preferably 0.02 to 8 wt.%, more preferably 0.05 to 6 wt.%, most preferably from 0.1 to 4 wt.%, based on the total weight of the cosmetic composition, is contained in the cosmetic composition.

5. A cosmetic composition for treating a keratinous material according to any one of claims 1 to 4, **characterized in that** the polyorganosiloxane is present in an amount of 0.1 to 30 wt.%, preferably 0.5 to 20 wt.%, more preferably 1 to 12 wt.%, even more preferably 1.5 to 9 wt.%, most preferably 2 to 6 wt.%, based on the total weight of the cosmetic composition, is contained in the cosmetic composition.

6. A cosmetic composition for treating keratinous material according to any one of claims 1 to 5, **characterized in that** the composition for treating keratinous material contains at least one organic silicon compound of formula (IV),
R₉Si(OR₁₀)ₖ(R₁₁)ₘ (IV),
which is selected from the group consisting of
- methyltrimethoxysilane
- methyltriethoxysilane
- ethyltrimethoxysilane
- ethyltriethoxysilane
- propyltriethoxysilane
- Propyltriethoxysilane
- Hexyltriethoxysilane
- Hexyltriethoxysilane
- Octyltrimethoxysilane
- Octyltriethoxysilane
- Dodecyltrimethoxysilane and
- Dodecyltriethoxysilane
- octadecyltriethoxysilane and
- octadecyltriethoxysilane.

7. A cosmetic composition for treating a keratinous material according to any one of claims 1 to 6, **characterized in that** the composition for treating a keratinous material - based on the total weight of the composition for treating a keratinous material - contains:
- 0.5 to 3% by weight of at least one first organic silicon compound selected from the group consisting of (3-aminopropyl)trimethoxysilane, (3-aminopropyl)triethoxysilane, (2-aminoethyl)trimethoxysilane, and (2-aminoethyl)triethoxysilane, and
- 3.2 to 7 wt% of at least one second organic silicon compound selected from the group consisting of methyltrimethoxysilane, methyltriethoxysilane, ethyltrimethoxysilane, ethyltriethoxysilane, propyltrimethoxysilane, propyltriethoxysilane, hexyltrimethoxysilane, hexyltriethoxysilane, octyltrimethoxysilane, octyltriethoxysilane, dodecyltrimethoxysilane, dodecyltriethoxysilane, octadecyltrimethoxysilane, and octadecyltriethoxysilane.

8. Use of a cosmetic composition for treating keratinous material according to any one of claims 1 to 7
for the care of keratinous material,
to reduce and/or prevent the harmful effects of air and water pollution on keratinous material, and/or
for reducing and/or preventing the fading of oxidatively colored keratinous material.

## Revendications

1. Produit cosmétique destiné au traitement d'une matière kératinique, comprenant
a) au moins un composé organique du silicium, qui contient au moins un composé organique du silicium de formule (I) et au moins un composé organique du silicium de formule (IV), dans lequel, dans le composé organique du silicium de formule (I)
R₁ R₂ N-L-Si(OR₃)ₐ(R₄)_{b} (I),
- R₁ et R₂ représentent tous deux un atome d'hydrogène,
- L représente un groupe alkylène linéaire à deux liaisons en C₁ -C₆,
- R₃ et R₄ représentent, indépendamment l'un de l'autre, un groupe méthyle ou un groupe éthyle,
- a représente le nombre 3 et
- b représente le nombre 0, et
dans lequel, dans le composé organique du silicium de formule (IV)
R₉ Si(OR₁₀)ₖ(R₁₁)ₘ (IV),
- R₉ représente un groupe alkyle en C₁-C₁₂,
- R₁₀ représente un atome d'hydrogène ou un groupe alkyle en C₁-C₆,
- R₁₁ représente un groupe alkyle en C₁-C₆
- k représente un nombre entier compris entre 1 et 3, et
- m représente le nombre entier 3 - k, et
b) au moins un polyorganosiloxane comprenant du PCA (acide pyrrolidone-carboxylique), de la diméthicone/amodiméthicone (INCI).

2. Produit cosmétique destiné au traitement d'une matière kératinique selon la revendication 1,
caractérisé en ce
en ce que ledit au moins un composé organique du silicium contient en outre un composé de formule (II),
dans lequel, dans le composé organique du silicium de formule (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R₅, R₅-, R_{5"}, R₆, R_{6'} et R_{6"} représentent, indépendamment les uns des autres, un groupe alkyle en C₁ à C₆
- A, A', A", A"' et Aʺʺ représentent, indépendamment les uns des autres, un groupe alkylène en C₁ à C₂₀ linéaire ou ramifié,
- R₇ et R₈ représentent, indépendamment l'un de l'autre, un atome d'hydrogène, un groupe alkyle enC₁ -C₆, un groupe hydroxy-C₁-C₆-alkyle, un groupe alcényle en C₂-C₆ , un groupe amino-C₁-C₆ -alkyle ou un groupement de formule (III)
- (A‴)-Si(R₆")_{d"}(OR₅")_{c"} (III),
- c représente un nombre entier compris entre 1 et 3,
- d représente le nombre entier 3 - c,
- c' représente un nombre entier compris entre 1 et 3,
- d' représente le nombre entier 3 - c',
- c" représente un nombre entier compris entre 1 et 3,
- d" représente le nombre entier 3 - c",
- e représente 0 ou 1,
- f représente 0 ou 1,
- g représente 0 ou 1,
- h représente 0 ou 1,
à condition qu'au moins l'un des radicaux parmi e, f, g et h soit différent de 0.

3. Produit cosmétique destiné au traitement d'une matière kératinique selon l'une des revendications 1 ou 2,
**caractérisé en ce**
**que** le produit destiné au traitement d'une matière kératinique contient au moins un composé organique du silicium de formule (I), choisi dans le groupe constitué par
- (3-aminopropyl)triméthoxysilane
- (3-aminopropyl)triéthoxysilane
- le (2-aminoéthyl)triméthoxysilane et
- le (2-aminoéthyl)triéthoxysilane.

4. Produit cosmétique destiné au traitement d'une matière kératinique selon l'une des revendications 1 à 3, **caractérisé en ce que** le composé organique du silicium de formule (I) est contenu dans le produit cosmétique en une quantité de 0,01 à 10 % en poids, de préférence de 0,02 à 8 % en poids, de manière encore plus préférée de 0,05 à 6 % en poids, et de préférence encore de 0,1 à 4 % en poids, par rapport au poids total du produit cosmétique, est contenue dans le produit cosmétique.

5. Produit cosmétique destiné au traitement d'une matière kératinique selon l'une des revendications 1 à 4, **caractérisé en ce que** le polyorganosiloxane est contenu dans le produit cosmétique en une quantité de 0,1 à 30 % en poids, de préférence de 0,5 à 20 % en poids, plus préférablement de 1 à 12 % en poids, encore plus préférablement de 1,5 à 9 % en poids, et de préférence encore de 2 à 6 % en poids, par rapport au poids total du produit cosmétique, est contenue dans le produit cosmétique.

6. Produit cosmétique destiné au traitement d'une matière kératinique selon l'une des revendications 1 à 5, **caractérisé en ce que** le produit destiné au traitement d'une matière kératinique contient au moins un composé organique du silicium de formule (IV),
R₉Si(OR₁₀)ₖ(R₁₁)ₘ (IV),
qui est choisi dans le groupe constitué par
- le méthyltriméthoxysilane
- le méthyltriéthoxysilane
- l'éthyltriméthoxysilane
- l'éthyltriéthoxysilane
- le propyltriéthoxysilane
- propyltriéthoxysilane
- Hexyltriéthoxysilane
- Hexyltriéthoxysilane
- octyltriméthoxysilane
- octyltriéthoxysilane
- dodécyltriméthoxysilane et
- dodécyltriéthoxysilane
- octadécyltriéthoxysilane et
- octadécyltriéthoxysilane.

7. Produit cosmétique destiné au traitement d'une matière kératinique selon l'une des revendications 1 à 6, **caractérisé en ce que** le produit destiné au traitement d'une matière kératinique contient, par rapport au poids total du produit destiné au traitement d'une matière kératinique :
- 0,5 à 3 % en poids d'au moins un premier composé organique du silicium choisi dans le groupe constitué par le (3-aminopropyl)triméthoxysilane, le (3-aminopropyl)triéthoxysilane, le (2-aminoéthyl)triméthoxysilane et le (2-aminoéthyl)triéthoxysilane, et
- 3,2 à 7 % en poids d'au moins un deuxième composé organique du silicium choisi dans le groupe constitué par le méthyltriméthoxysilane, le méthyltriéthoxysilane, l'éthyltriméthoxysilane, éthyltriéthoxysilane, propyltriméthoxysilane, propyltriéthoxysilane, hexyltriméthoxysilane, hexyltriéthoxysilane, octyltriméthoxysilane, octyltriéthoxysilane, le dodécyltriméthoxysilane, le dodécyltriéthoxysilane, l'octadécyltriméthoxysilane et l'octadécyltriéthoxysilane.

8. Utilisation d'un produit cosmétique pour le traitement d'une matière kératinique selon l'une des revendications 1 à 7
pour le soin d'une matière kératinique,
pour réduire et/ou prévenir les effets néfastes de la pollution atmosphérique et de la pollution de l'eau sur la matière kératinique et/ou
pour réduire et/ou empêcher la décoloration d'une matière kératinique colorée par oxydation.
